# EUROPÄISCHE PATENTSCHRIFT

(11) **EP 0 854 871 B1**
(45) Veröffentlichungstag und Bekanntmachung des Hinweises auf die Patenterteilung: **16.08.2000**
(21) Anmeldenummer: 96930054.0
(22) Anmeldetag: 19.08.1996
(51) Int. Cl.: C07D 271/113, A01N 43/824

(54) **1,3,4-OXADIAZOLIN-2-ONE UND IHRE VERWENDUNG ALS SCHÄDLINGSBEKÄMPFUNGSMITTEL**
1,3,4-OXADIAZOLIN-2-ONES AND THEIR USE AS PESTICIDES
1,3,4-OXADIAZOLIN-2-ONES ET LEUR UTILISATION COMME PESTICIDES

(30) Priorität: 30.08.1995 DE 19531892
(43) Veröffentlichungstag der Anmeldung: 29.07.1998
(73) Patentinhaber: BAYER AG, 51368 Leverkusen (DE)
(72) Erfinder: KLEEFELD, Gerd, D-41468 Neuss (DE); WACHENDORFF-NEUMANN, Ulrike, D-56566 Neuwied (DE)
(86) Internationale Anmeldenummer: EP9603640
(87) Internationale Veröffentlichungsnummer: WO9708158

(56) Entgegenhaltungen:
- EP-A- 0 270 061
- DE-A- 3 631 511

## Beschreibung

Die vorliegende Erfindung betrifft neue 1,3,4-Oxadiazolin-2-one, Verfahren zu ihrer Herstellung und ihre Verwendung zur Bekämpfung von tierischen Schädlingen.

Es ist bereits bekannt, daß bestimmte, substituierte 1,3,4-Oxa(thia)diazolin-2-one insektizide und akarizide Eigenschaften aufweisen (vgl. EP-A 0 270 061). Die Wirkungshöhe und/oder Wirkungsdauer dieser vorbekannten Verbindungen ist jedoch, insbesondere bei niedrigen Aufwandmengen und Konzentrationen nicht immer in allen Anwendungsgebieten zufriedenstellend.

Es wurden neue 1,3,4-Oxadiazolin-2-one der Formel (I) 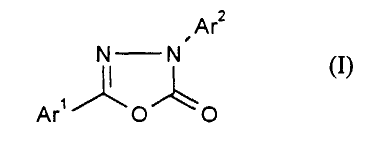 gefunden, in welcher
- Ar¹: für gegebenenfalls substituiertes Phenyl steht und
- Ar²: für gegebenenfalls substituiertes Biphenyl steht.

Weiterhin wurde gefunden, daß man 1,3,4-Oxadiazolin-2-one der Formel (I) erhält, wenn man
a) Hydrazide der Formel (II) 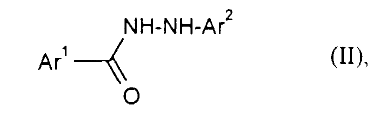 in welcher
   - Ar¹ und Ar²: die oben angegebene Bedeutung haben,
   mit einem Acylierungsmitttel in Gegenwart eines Verdünnungsmittels und gegebenenfalls in Gegenwart einer Base umsetzt,
   oder
b) 3-Phenyl-1,3,4-oxadiazolin-2-one der Formel (III) 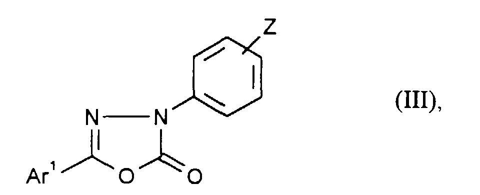 in welcher
   - Ar¹: die oben angegebene Bedeutung hat und
   - Z: für eine Abgangsgruppe steht,
   mit Boronsäuren der Formel (IV)

   R-B(OH)₂ (IV),

   in welcher
   - R: für gegebenenfalls substituiertes Phenyl steht,
   in Gegenwart einer Base, gegebenenfalls in Gegenwart eines Katalysators und in Gegenwart eines Verdünnungsmittels umsetzt.

Weiterhin wurde gefunden, daß die neuen 1,3,4-Oxadiazolin-2-one der Formel (I) sehr gut zur Bekämpfung von tierischen Schädlingen geeignet sind. Sie zeichnen sich insbesondere durch hohe Wirksamkeit gegen Arthropoden aus.

Die erfindungsgemäßen 1,3,4-Oxadiazolin-2-one sind durch die Formel (I) allgemein definiert.

Bevorzugte Substituenten bzw. Bereiche der in den oben und nachstehend erwähnten Formeln aufgeführten Reste werden im folgenden erläutert.
- Ar¹: steht vorzugsweise für Phenyl, das gegebenenfalls einfach bis fünffach, gleich oder verschieden substituiert ist durch Halogen, C₁-C₆-AJkyl, C₁-C₆-Alkoxy, C₁-C₆-Alkylthio, C₁-C₆-Halogenalkyl, C₁-C₆-Halogenalkoxy, C₁-C₆-Halogenalkylthio, Nitro oder Cyano.
- Ar²: steht vorzugsweise für den Rest 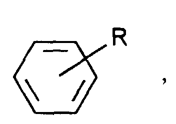 worin
- R: für gegebenenfalls einfach bis fünffach, gleich oder verschieden durch CHO, Cyano, Halogen, C₁-C₁₂-Alkyl, C₁-C₄-Halogenalkyl, C₁-C₁₂-Alkoxy, C₁-C₆-Halogenalkoxy, C₁-C₆-Alkoxy-C₁-C₆-alkyl, C₁-C₆-Alkoxyethylenoxy, C₁-C₆-Alkylthio oder C₁-C₆-Halogenalkylthio substituiertes Phenyl steht.
- Ar¹: steht besonders bevorzugt für Phenyl, das gegebenenfalls einfach bis dreifach, gleich oder verschieden substituiert ist durch Fluor, Chlor, Brom, C₁-C₄-Alkyl, C₁-C₄-Alkoxy, C₁-C₄-Alkylthio; einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro oder Cyano.
- Ar²: steht besonders bevorzugt für den Rest 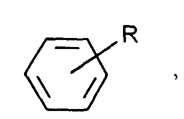 worin
- R: für gegebenenfalls einfach bis fünffach, gleich oder verschieden durch CHO, Cyano, Fluor, Chlor, Brom, C₁-C₁₂-Alkyl, einfach bis sechsfach, gleich oder verschieden durch Fluor oder Chlor substituiertes C₁-C₄-Alkyl, C₁-C₁₂-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxy-ethylenoxy, C₁-C₄-Alkylthio oder einfach bis sechsfach, gleich oder verschieden durch Fluor oder Chlor substituiertes C₁-C₄-Alkylthio substituiertes Phenyl steht.
- Ar¹: steht ganz besonders bevorzugt für einfach bis dreifach, gleich oder verschieden durch Fluor oder Chlor substituiertes Phenyl.
- Ar²: steht besonders bevorzugt für den Rest 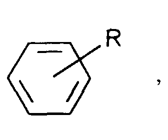 worin
- R: für gegebenenfalls einfach bis fünffach, gleich oder verschieden durch CHO, Cyano, Fluor, Chlor, Brom, C₁-C₁₂-Alkyl, einfach bis sechsfach, gleich oder verschieden durch Fluor oder Chlor substituiertes C₁-C₄-Alkyl, C₁-C₁₂-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxy-ethylenoxy, C₁-C₄-Alkylthio oder einfach bis sechsfach, gleich oder verschieden durch Fluor oder Chlor substituiertes C₁-C₄-Alkylthio substituiertes Phenyl steht.
- Ar¹: steht besonders hervorgehoben für einfach oder zweifach, gleich oder verschieden durch Fluor oder Chlor substituiertes Phenyl.
- Ar²: steht besonders hervorgehoben für den Rest 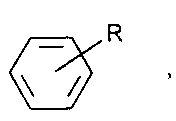 worin
- R: für gegebenenfalls einfach bis dreifach, gleich oder verschieden durch CHO, Cyano, Fluor, Chlor, Brom, C₁-C₈-Alkyl, einfach bis sechsfach, gleich oder verschieden durch Fluor oder Chlor substituiertes C₁-C₄-Alkyl, C₁-C₈-Alkoxy, jeweils einfach bis fünffach, gleich oder verschieden durch Fluor oder Chlor substituiertes Methoxy oder Ethoxy, C₁-C₄-AJkoxy- C₁-C₄-alkyl, C₁-C₄-Alkylthio, jeweils einfach bis fünffach, gleich oder verschieden durch Fluor oder Chlor substituiertes Methylthio oder Ethylthio substituiertes Phenyl steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß besonders hervorgehoben werden die Verbindungen der Formel (I), in welchen eine Kombination der vorstehend als besonders hervorgehobenen aufgeführten Bedeutungen vorliegt.

In den oben und nachstehend aufgeführten Restedefinitionen sind Kohlenwasserstoffreste, wie Alkyl - auch in Verbindungen mit Heteroatomen wie Alkoxy oder Alkylthio - soweit möglich jeweils geradkettig oder verzweigt.

Bevorzugte erfindungsgemäße Verbindungen sind Stoffe der Formel (IA): 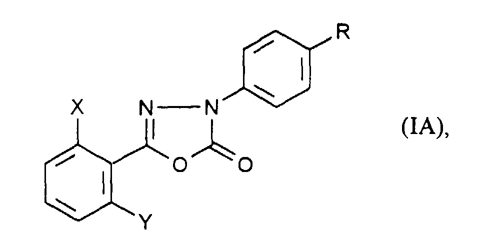 in welcher
- X: für H, F oder Cl steht,
- Y: für F oder Cl steht und
- R: die oben genannte Bedeutung hat.

Beispiele für die neuen erfindungsgemäßen Verbindungen sind in den Tabellen 1 bis 5 aufgeführt.

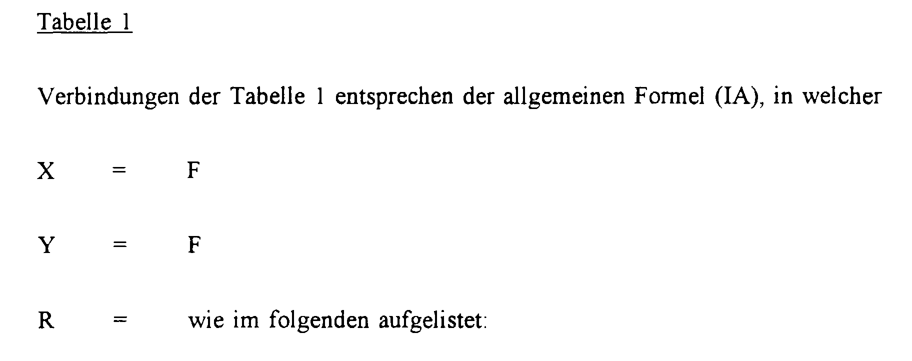

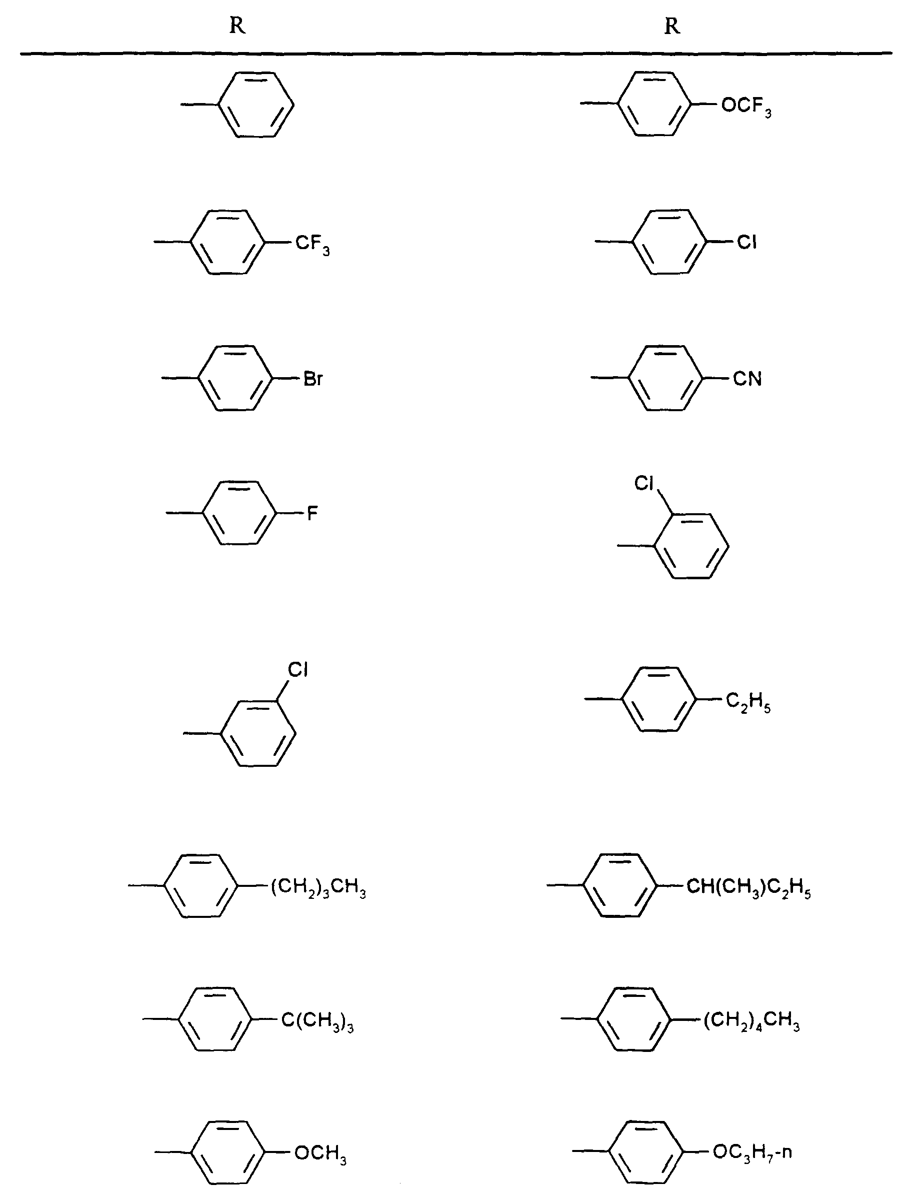

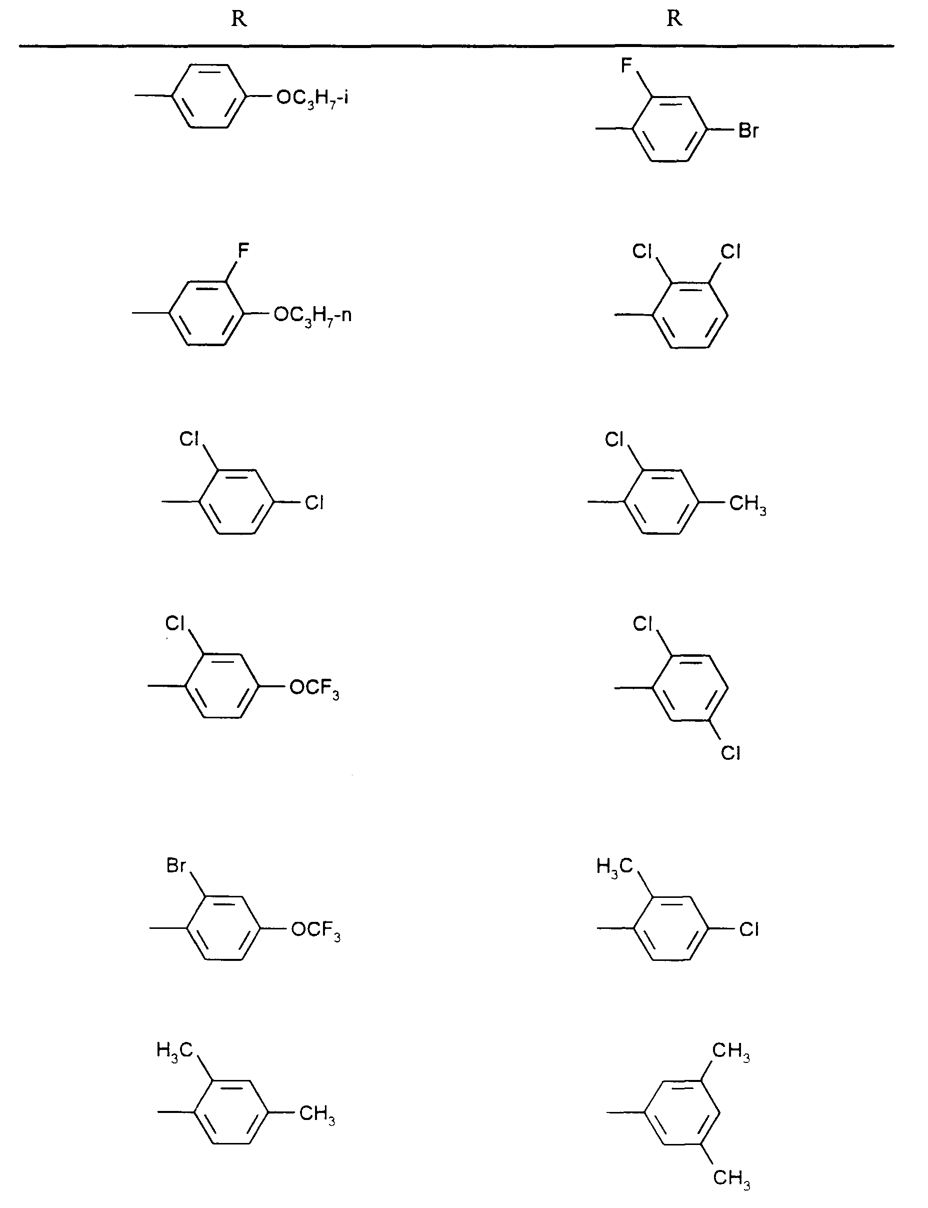

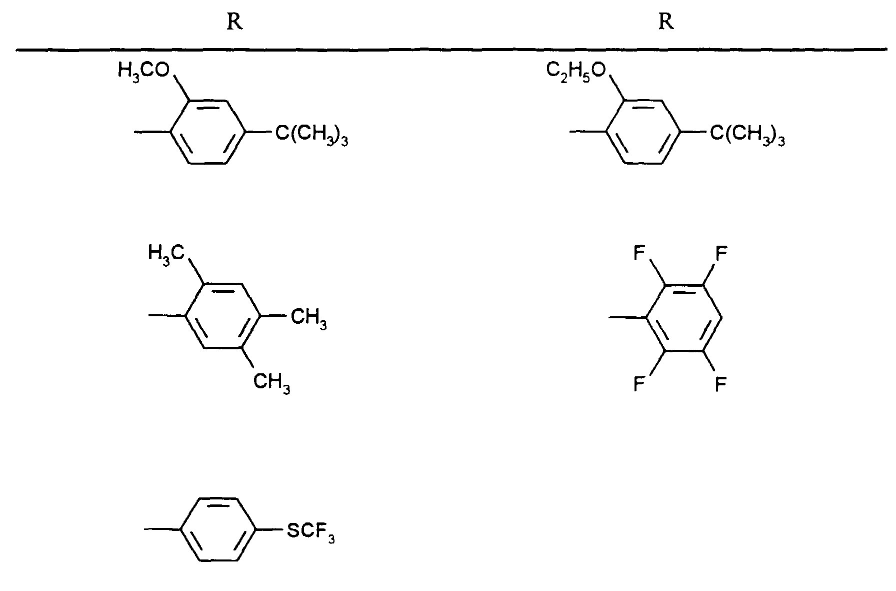

**Tabelle 2**

| Verbindungen der Tabelle 2 entsprechen der allgemeinen Formel (IA), in welcher | |
|---|---|
| X = | H |
| Y = | F |
| R = | wie in Tabelle 1 aufgelistet. |

**Tabelle 3**

| Verbindungen der Tabelle 3 entsprechen der allgemeinen Formel (IA), in welcher | |
|---|---|
| X = | H |
| Y = | Cl |
| R = | wie in Tabelle 1 aufgelistet. |

**Tabelle 4**

| Verbindungen der Tabelle 4 entsprechen der allgemeinen Formel (IA), in welcher | |
|---|---|
| X = | F |
| Y = | Cl |
| R = | wie in Tabelle 1 aufgelistet. |

**Tabelle 5**

| Verbindungen der Tabelle 5 entsprechen der allgemeinen Formel (IA), in welcher | |
|---|---|
| X = | Cl |
| Y = | Cl |
| R = | wie in Tabelle 1 aufgelistet. |

Verwendet man beispielsweise N'-[4-(4-Trifluormethoxyphenyl)phenyl]-2,6-difluorbenzoesäurehydrazid als Ausgangsstoff und Chlorameisensäuretrichlormethylester als Acylierungsmittel, so kann der Verlauf des erfindungsgemäßen Verfahrens (a) durch das folgende Reaktionsschema wiedergegeben werden:

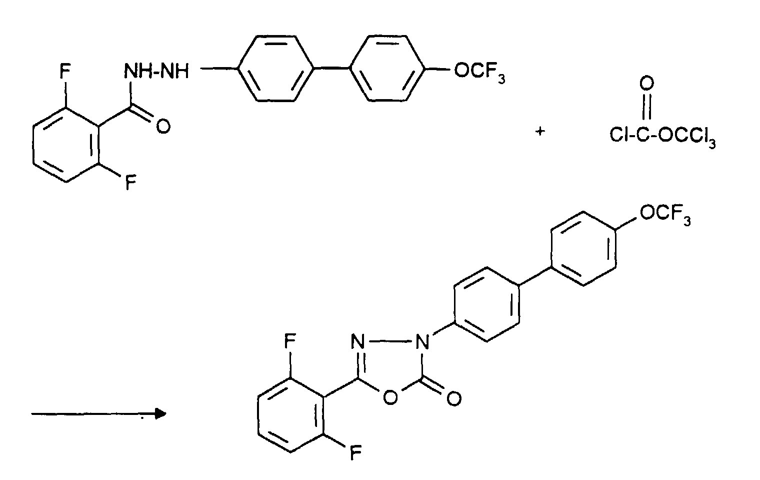

Verwendet man beispielsweise 3-[4-(4-Bromphenyl)-phenyl]-5-(2,6-difluorphenyl)-1,3,4-oxadiazolin-2-on und 4-Trifluormethoxyphenylboronsäure als Ausgangsstoffe, so kann der Verlauf des erfindungsgemäßen Verfahrens (b) durch das folgende Reaktionsschema wiedergegeben werden:

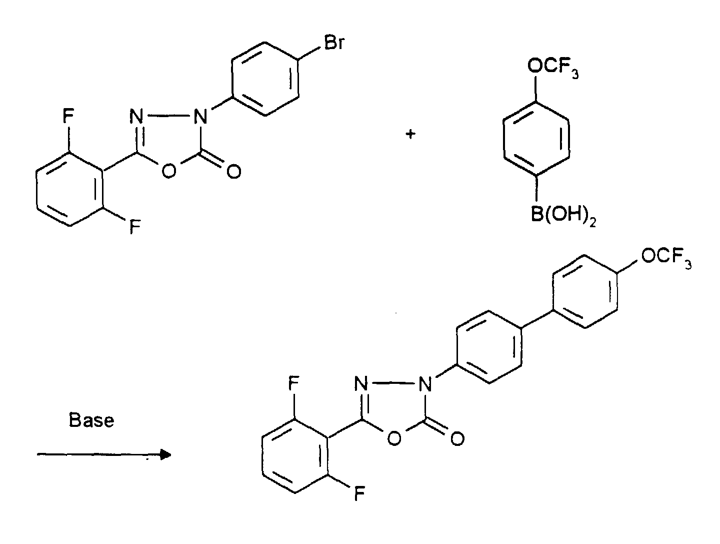

Die beim erfindungsgemäßen Verfahren (a) als Ausgangsstoffe zu verwendenden Hydrazide sind durch die Formel (II) allgemein definiert.

Die Hydrazide der Formel (II) sind bekannt bzw. können in allgemein bekannter Art und Weise erhalten werden (vgl. hierzu z.B. US 2 167 793; Revue Roumaine de Chimie 29 (2), 219 - 22 (1984); Liebigs Annalen der Chemie 1975, 1264). Man erhält Hydrazide der Formel (II) beispielsweise, wenn man aktive Carbonsäurederivate, beispielsweise der Formeln (Va-c)

Ar¹―CO―Cl (Va),

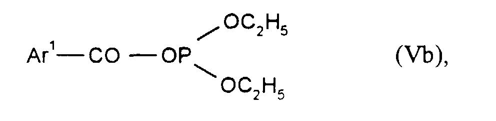

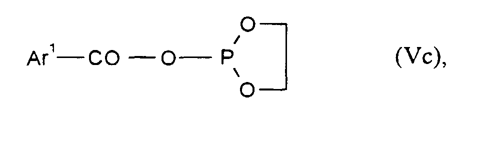

in welchen
- Ar¹: die oben angegebene Bedeutung hat,
mit Hydrazinen der Formel (VI)

Ar²-NH-NH₂ (VI),

in welcher
- Ar²: die oben angegebene Bedeutung hat,
in Gegenwart eines Verdünnungsmittels, z.B. eines gegebenenfalls halogenierten, aliphatischen oder aromatischen Kohlenwasserstoffs wie beispielsweise Methylenchlorid oder Toluol, und gegebenenfalls in Gegenwart einer Base, z.B. einer organischen Stickstoffbase, wie beispielsweise Triethylamin, bei Temperaturen zwischen 0° und 100°C umsetzt (vgl auch US 2 617 793; Revue Roumaine de Chimie 29 (2), 219 - 22 (1984));
oder wenn man Hydrazide der Formel (IIa) 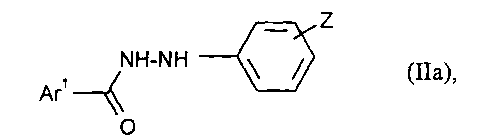 in welcher
- Ar¹ und Z: die oben angegebene Bedeutung haben,
mit Boronsäuren der Formel (IV) gemäß dem erfindungsgemäßen Verfahren (b) umsetzt (vgl. auch Comprehensive Organic Synthesis, Vol. III, S. 435-520 (1992) und die Herstellungsbeispiele).

Die Hydrazide der Formel (IIa) sind bekannt oder in einfacher Weise mit Hilfe bekannter Methoden erhältlich. Man erhält sie beispielsweise, wenn man aktivierte Carbonsäurederivate der Formeln (Va-c) mit Hydrazinen der Formel (VIa) 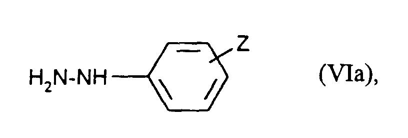 in welcher
- Z: die oben angegebene Bedeutung hat,
wie bereits oben beschrieben, umsetzt.

Die Carbonsäurederivate der Formeln (Va-c) sowie die Hydrazine der Formeln (VI) und (VIa) sind bekannt und/oder können in allgemein bekannter Art und Weise erhalten werden.

Für das erfindungsgemäße Verfahren (a) werden weiterhin Acylierungsmittel als Ausgangsstoffe benötigt. Hierzu gehören vorzugsweise Chlorameisensäureester, wie insbesondere Chlorameisensäure-trichlormethylester, Phosgen sowie Chlorameisensäure-methylester oder -ethylester.

Die Acylierungsmittel sind allgemein bekannte Verbindungen der organischen Chemie.

Die beim erfindungsgemäßen Verfahren (b) als Ausgangsstoffe zu verwendenden 3-Phenyl-l,3,4-oxadiazolin-2-one sind durch die Formel (III) allgemein definiert.

Z steht vorzugsweise für Halogen oder -OSO₂CF₃, besonders bevorzugt für Brom oder Iod.

Die 3-Phenyl-1,3,4-oxadiazolin-2-one der Formel (III) sind teilweise bekannt (vgl. z.B. EP-A 0 270 061). Noch nicht bekannt und ebenfalls Gegenstand der vorliegenden Erfindung sind 3-Phenyl-1,3,4-oxadiazolin-2-one der Formel (IIIa) 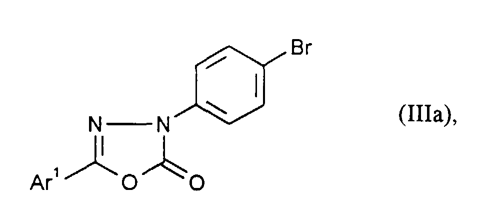 in welcher
- Ar¹: die oben angegebene Bedeutung hat.

Die 3-Phenyl-1,3,4-oxadiazolin-2-one der Formel (III), einschließlich der neuen Verbindungen der Formel (IIIa) können gemäß dem erfindungsgemäßen Verfahren (a) erhalten werden.

Die außerdem zur Durchführung des erfindungsgemäßen Verfahrens (b) als Ausgangsstoffe benötigten Boronsäuren sind durch die Formel (IV) allgemein definiert.

Die Boronsäuren der Formel (IV) sind allgemein bekannte Verbindungen der organischen Chemie.

Als Verdünnungsmittel kommen bei der Durchführung der erfindungsgemäßen Verfahren (a) und (b) alle unter den jeweils gegebenen Reaktionsbedingungen inerten organischen Lösungsmittel infrage. Sie können gegebenenfalls in Mischung mit Wasser verwendet werden. Bevorzugt verwendet werden Kohlenwasserstoffe wie Toluol, Xylol, Tetralin, Hexan, Cyclohexan, Halogenkohlenwasserstoffe wie Methylenchlorid, Chloroform, Chlorbenzol, o-Dichlorbenzol, Ether wie Diethylether, Diisopropylether, Dimethoxyethan, Tetrahydrofuran, Dioxan, Nitrile wie Acetonitril oder Butyronitril, Amide wie Dimethylformamid, ferner Sulfolan.

Als Base kommen bei der Durchführung der erfindungsgemäßen Verfahren (a) und (b) alle üblichen Säureakzeptoren infrage. Vorzugsweise verwendbar sind tertiäre Amine wie Triethylamin, Pyridin, Diazabicyclooctan (DABCO), Diazabicycloundecen (DBU), Diazabicyclononen (DBN), N,N-Dimethylanilin, ferner Erdalkalimetalloxide wie Magnesium- oder Calciumoxid, außerdem Alkali- oder Erdalkalimetallcarbonate wie Natriumcarbonat, Kaliumcarbonat oder Calciumcarbonat, Alkalihydroxide wie Natrium- oder Kaliumhydroxid, ferner Alkoholate wie Natriumethanolat oder Kalium-tert.-butylat.

Als Katalysatoren kommen bei der Durchführung des erfindungsgemäßen Verfahrens (b) Palladium oder seine Verbindungen bzw. Komplexe, vorzugsweise Palladium oder Tetrakis-(triphenylphosphin)-palladium infrage.

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0°C und 150°C, bevorzugt zwischen 0°C und 100°C, beziehungsweise bei der Siedetemperatur des verwendeten Lösungsmittels.

Bei der Durchführung des erfindungsgemäßen Verfahrens (a) setzt man auf 1 Mol Hydrazid der Formel (II) 1 bis 5 Mol, vorzugsweise 1 bis 2,5 Mol Acylierungsmittel und gegebenenfalls 1 bis 5 Mol, vorzugsweise 1 bis 2,5 Mol Base ein

Dabei ist es auch möglich, die Reaktion zweistufig ablaufen zu lassen, d.h. zunächst Addition des Acylierungsmittels und anschließende Cyclisierung durch Erhitzen (vgl.. hierzu auch EP-A 0 270 061).

Bei der Durchführung des erfindungsgemäßen Verfahrens (b) arbeitet man allgemein in äquimolaren Mengen, wobei gegebenenfalls 0,01 bis 0,1 Mol an Katalysator sowie 1 bis 5 Mol an Base eingesetzt werden.

Die Aufarbeitung und Isolierung der Endprodukte erfolgt in allgemein üblicher Art und Weise.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp, Trogoderma spp, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata. Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp. Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (I) zeichnen sich insbesondere durch eine hohe akarizide Wirksamkeit aus.

Sie lassen sich mit besonders gutem Erfolg zur Bekämpfung von pflanzenschädigenden Milben, wie beispielsweise gegen die Bohnenspinnmilbe (Tetranychus urticae) einsetzen.

Die Wirkstoffe können in die üblichen Formulierungen übergeführt werden, wie Lösungen, Emulsionen, Spritzpulver, Suspensionen, Pulver, Stäubemittel, Pasten. lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie PolyoxyethylenFettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
Fungizide:
2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl)-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Außerdem wurde gefunden, daß die erfindungsgemäßen Verbindungen der Formel 1 eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus

Hautflügler wie
Sirex juvencus, Urocerus gigas, Urocerus gigas taignus, Urocerus augur

Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungsbzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise -Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, genannt.

Die Herstellung und die Verwendung der erfindungsgemäßen Wirkstoffe gehen aus den nachfolgenden Beispielen hervor.

### Beispiel 1

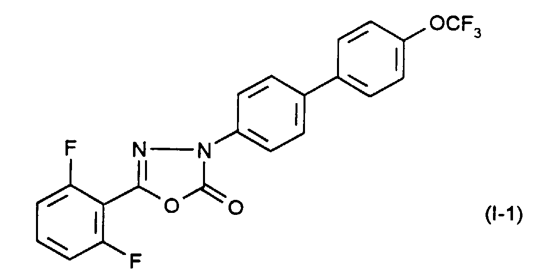

und 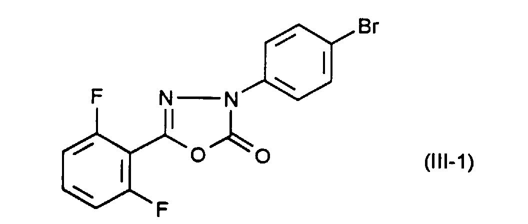 0,7 g (1.7 mMol) rohes (vgl. die Herstellung des Ausgangsproduktes) N'-[4-(4-Trifluormethoxyphenyl)phenyl]-2,6-difluorbenzoesäurehydrazid werden mit 0,67 g (3,4 mMol) Chlorameisensäuretrichlormethylester in 2 ml Toluol 0,5 Stunden bei Raumtemperatur und anschließend über Nacht bei 65°C (Badtemperatur) gerührt. Das Reaktionsgemisch wird auf Puffer (pH = 7) gegeben und mit Ether extrahiert. Die organische Phase wird über Natriumsulfat getrocknet, eingedampft und über HPLC aufgetrennt.

Man erhält 128 mg 5-(2,6-Difluorphenyl)-3-[4-(4-trifluormethoxyphenyl)phenyl]-1,3,4-oxadiazolin-2-on (I-1)
¹HNMR (ppm, in CDCl₃): 7.10 (t, 2H); 7.31 (d, 2H); 7.55 (dt, 1H); 7.62 (d, 2H); 7.67 (d, 2H); 8.03 (d, 2H),
und 225 mg 3-(4-Bromphenyl)-5-(2,6-difluorphenyl)-1,3,4-oxadiazolin-2-on (III-1) ¹HNMR (ppm, in CDCl₃): 7.10 (t, 2H); 7.54 (dt, 1H); 7.59 (d, 2H); 7.85 (d, 2H).

### Herstellung des Ausgangsproduktes

### Beispiel (II-1)

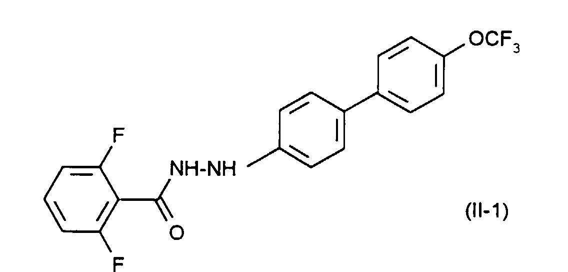

0,65 g (2 mMol) N'-(4-Bromphenyl)-2,6-difluorbenzoesäurehydrazid und 0,48 g 4-Trifluormethoxyboronsäure (wasserfeucht) werden mit 0,62 Kaliumcarbonat und einer Spatelspitze Tetrakis(triphenylphosphin)-palladium in 5 ml Lösungsmittelgemisch (Wasser/Toluol/Ethanol = 1:1:0,5) über Nacht bei 100°C gerührt. Zur Aufarbeitung gibt man auf Wasser und extrahiert mit Essigester. Die organische Phase wird mit gesättigter NaCl-Lösung neutralgewaschen, über Natriumsulfat getrocknet und anschließend eingedampft.

Man erhält 0,9 g N'[4-(4-Trifluormethoxyphenyl)phenyl]-2,6-difluorbenzoesäurehydrazid, das als Rohprodukt direkt weiter umgesetzt wird.

### Beispiel (IIa-1)

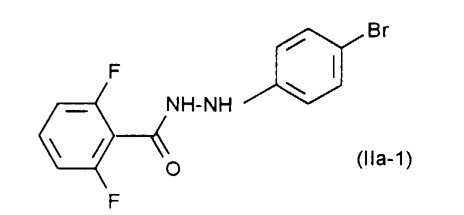

23,3 g (0,10 Mol) 4-Bromphenylhydrazinhydrochlorid werden mit 20,5 (0,2 Mol) Triethylamin in 350 ml Chloroform vorgelegt. Anschließend tropft man bei 10°C 17,6 g (0,1 Mol) 2,6-Difluorbenzoylchlorid zu und rührt über Nacht bei Raumtemperatur. Zur Aufarbeitung wird je einmal mit 10%iger Natronlauge, verdünnter Salzsäure und Wasser gewaschen. Die organische Phase wird über Natriumsulfat getrocknet und anschließend eingedampft.

Man erhält 14,7 g (45% der Theorie) N'-(4-Bromphenyl)-2,6-difluorbenzoesäurehydrazid vom Schmelzpunkt 172°C.

Entsprechend Beispiel 1 und gemäß den allgemeinen Angaben zur Herstellung werden die folgenden Verbindungen der Formel (I) erhalten:

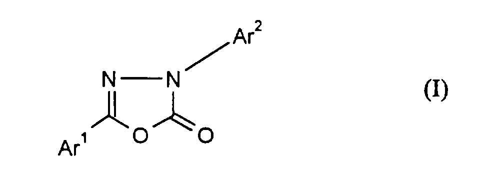

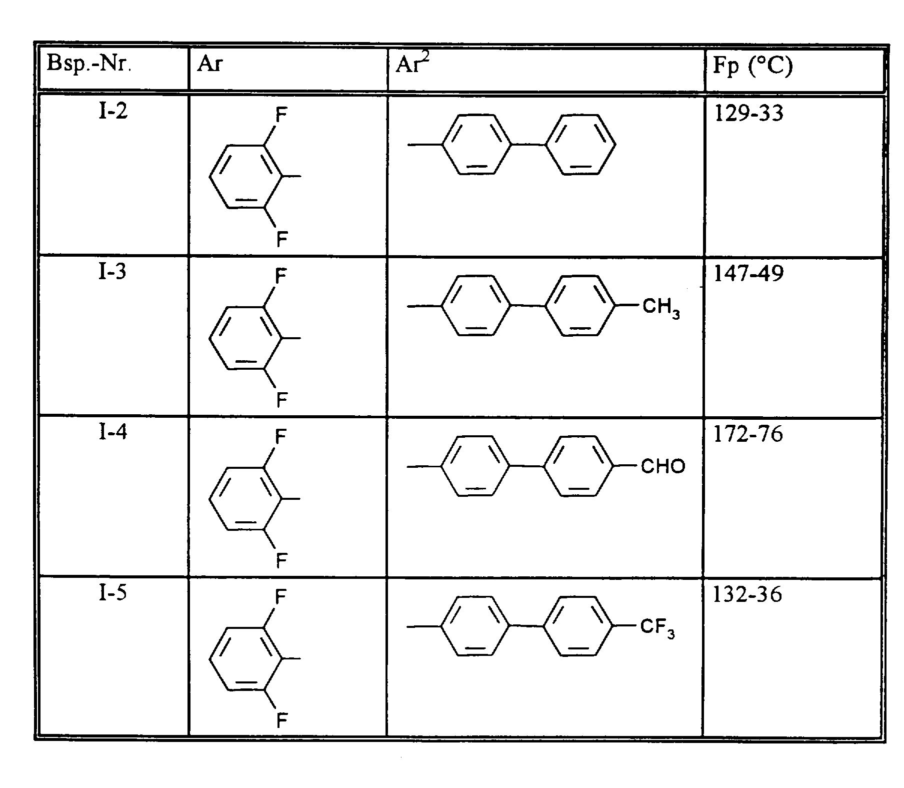

### Anwendungsbeispiele

### Beispiel A

**Tetranychus-Test** (OP-resistent/Tauchbehandlung)
- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe Tetranychus urticae befallen sind, werden in eine Wirkstoffzubereitung der gewünschten Konzentration getaucht.

Nach der gewünschten Zeit wird die Wirkung in % bestimmt. Dabei bedeutet 100%, daß alle Spinnmilben abgetötet wurden; 0% bedeutet, daß keine Spinnmilben abgetötet wurden.

In diesem Test bewirkt z.B. die Verbindung (I-1) gemäß Herstellungsbeispiel 1 bei einer beispielhaften Wirkstoffkonzentration von 0,1% eine Abtötung von 98% nach 13 Tagen.

## Patentansprüche

1. Verbindungen der Formel (I) 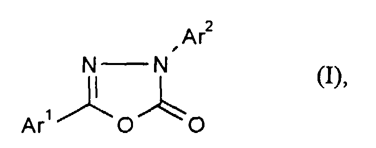 in welcher
Ar¹ für gegebenenfalls substituiertes Phenyl steht und
Ar² für gegebenenfalls substituiertes Biphenyl steht.

2. Verbindungen der Formel (I) gemäß Anspruch 1, in welcher
Ar¹ für Phenyl steht, das gegebenenfalls einfach bis fünffach, gleich oder verschieden substituiert ist durch Halogen, C₁-C₆-Alkyl, C₁-C₆-Alkoxy, C₁-C₆-Alkylthio, C₁-C₆-Halogenalkyl, C₁-C₆-Halogenalkoxy, C₁-C₆-Halogenalkylthio, Nitro oder Cyano und
Ar² für den Rest 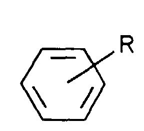 steht, worin
R für gegebenenfalls einfach bis fünffach, gleich oder verschieden durch CHO, Cyano, Halogen, C₁-C₁₂-Alkyl, C₁-C₄-Halogenalkyl, C₁-C₁₂-Alkoxy, C₁-C₆-Halogenalkoxy, C₁-C₆-Alkoxy-C₁-C₆-alkyl, C₁-C₆-Alkoxy-ethylenoxy, C₁-C₆-Alkylthio oder C₁-C₆-Halogenalkylthio substituiertes Phenyl steht.

3. Verbindungen der Formel (I) gemäß Anspruch 1, in welcher
Ar¹ für Phenyl steht, das gegebenenfalls einfach bis dreifach, gleich oder verschieden substituiert ist durch Fluor, Chlor, Brom, C₁-C₄-Alkyl, C₁-C₄-Alkoxy, C₁-C₄-Alkylthio; einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro oder Cyano und
Ar² für den Rest 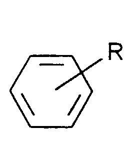 steht , worin
R für gegebenenfalls einfach bis fünffach, gleich oder verschieden durch CHO, Cyano, Fluor, Chlor, Brom, C₁-C₁₂-Alkyl, einfach bis sechsfach, gleich oder verschieden durch Fluor oder Chlor substituiertes C₁-C₄-AJkyl, C₁-C₁₂-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxy-ethylenoxy, C₁-C₄-Alkylthio oder einfach bis sechsfach, gleich oder verschieden durch Fluor oder Chlor substituiertes C₁-C₄-Alkylthio substituiertes Phenyl steht.

4. Verbindungen der Formel (I) gemäß Anspruch 1, in welcher
Ar¹ für einfach bis dreifach, gleich oder verschieden durch Fluor oder Chlor substituiertes Phenyl steht und
Ar² für den Rest 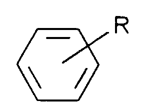 steht, worin
R für gegebenenfalls einfach bis fünffach, gleich oder verschieden durch CHO, Cyano, Fluor, Chlor, Brom, C₁-C₁₂-Alkyl, einfach bis sechsfach, gleich oder verschieden durch Fluor oder Chlor substituiertes C₁-C₄-Alkyl, C₁-C₁₂-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxy-ethylenoxy, C₁-C₄-Alkylthio oder einfach bis sechsfach, gleich oder verschieden durch Fluor oder Chlor substituiertes C₁-C₄-Alkylthio substituiertes Phenyl steht

5. Verbindungen der Formel (I) gemäß Anspruch 1, in welcher
Ar¹ für einfach oder zweifach, gleich oder verschieden durch Fluor oder Chlor substituiertes Phenyl steht und
Ar² für den Rest 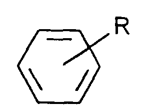 steht, worin
R für gegebenenfalls einfach bis dreifach, gleich oder verschieden durch CHO, Cyano, Fluor, Chlor, Brom, C₁-C₈-Alkyl, einfach bis sechsfach, gleich oder verschieden durch Fluor oder Chlor substituiertes C₁-C₄-Alkyl, C₁-C₈-Alkoxy, jeweils einfach bis fünffach, gleich oder verschieden durch Fluor oder Chlor substituiertes Methoxy oder Ethoxy, C₁-C₄-Alkoxy- C₁-C₄-alkyl, C₁-C₄-Alkylthio, jeweils einfach bis fünffach, gleich oder verschieden durch Fluor oder Chlor substituiertes Methylthio oder Ethylthio substituiertes Phenyl steht.

6. Verfahren zur Herstellung von Verbindungen der Formel (I) gemäß Anspruch 1, dadurch gekennzeichnet, daß man
a) Hydrazide der Formel (II) 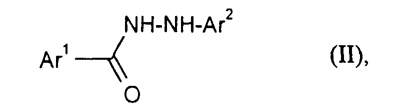 in welcher
Ar¹ und Ar² die in Anspruch 1 angegebene Bedeutung haben,
mit einem Acylierungsmitttel in Gegenwart eines Verdünnungsmittels und gegebenenfalls in Gegenwart einer Base umsetzt,
oder
b) 3-Phenyl-1,3,4-oxadiazolin-2-one der Formel (III) 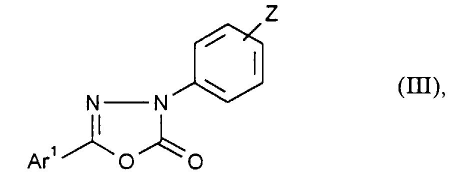 in welcher
Ar¹ die oben angegebene Bedeutung hat und
Z für eine Abgangsgruppe steht,
mit Boronsäuren der Formel (IV)
R-B-(OH)₂ (IV),
in welcher
R für gegebenenfalls substituiertes Phenyl steht,
in Gegenwart einer Base, gegebenenfalls in Gegenwart eines Katalysators und in Gegenwart eines Verdünnungsmittels umsetzt.

7. Verbindungen der Formel (IIIa) 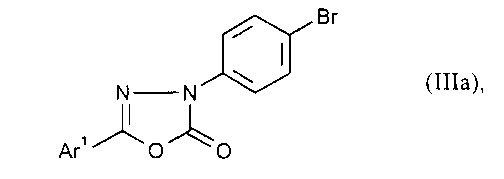 in welcher
Ar¹ die in Anspruch 1 angegebene Bedeutung hat.

8. Schädlingsbekämpfungsmittel, gekennzeichnet durch einen Gehalt an mindestens einer Verbindung der Formel (I) gemäß Anspruch 1.

9. Verwendung von Verbindungen der Formel (I) gemäß Anspruch 1 zur Bekämpfung von Schädlingen.

10. Verfahren zur Bekämpfung von Schädlingen, dadurch gekennzeichnet, daß man Verbindungen der Formel (I) gemäß Anspruch 1 auf Schädlinge und/oder ihren Lebensraum einwirken läßt.

11. Verfahren zur Herstellung von Schädlingsbekämpfungsmitteln, dadurch gekennzeichnet, daß man Verbindungen der Formel (I) gemäß Anspruch 1 mit Streckmitteln und/oder oberflächenaktiven Mitteln vermischt.

12. Verwendung von Verbindungen der Formel (I) gemäß Anspruch 1 zur Herstellung von Schädlingsbekämpfungsmitteln.

## Claims

1. Compounds of the formula (I) 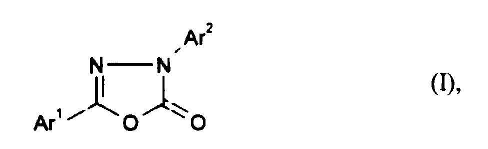 in which
Ar¹ represents optionally substituted phenyl and
Ar² represents optionally substituted biphenyl.

2. Compounds of the formula (I) according to Claim 1, in which
Ar¹ represents phenyl which is optionally mono- to pentasubstituted by identical or different substituents from the group consisting of halogen, C₁-C₆-alkyl, C₁-C₆-alkoxy, C₁-C₆-alkylthio, C₁-C₆-halogenoalkyl, C₁-C₆-halogenoalkoxy, C₁-C₆-halogenoalkylthio, nitro and cyano and
Ar² represents the radical 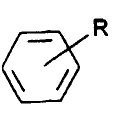 in which
R represents phenyl which is optionally mono- to pentasubstituted by identical or different substituents from the group consisting of CHO, cyano, halogen, C₁-C₁₂-alkyl, C₁-C₄-halogenoalkyl, C₁-C₁₂-alkoxy, C₁-C₆-halogenoalkoxy, C₁-C₆-alkoxy-C₁-C₆-alkyl, C₁-C₆-alkoxy-ethyleneoxy, C₁-C₆-alkylthio and C₁-C₆-halogenoalkylthio.

3. Compounds of the formula (I) according to Claim 1 in which
Ar¹ represents phenyl which is optionally mono- to trisubstituted by identical or different substituents from the group consisting of fluorine, chlorine, bromine, C₁-C₄-alkyl, C₁-C₄-alkoxy, C₁-C₄-alkylthio; C₁-C₂-alkyl which is mono- to pentasubstituted by identical or different substituents from the group consisting of fluorine and chlorine, C₁-C₄-alkoxy which is mono- to pentasubstituted by identical or different substituents from the group consisting of fluorine and chlorine, SCF₃, SCHF₂, nitro and cyano and
Ar² represents the radical 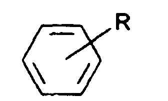 in which
R represents phenyl which is optionally mono- to pentasubstituted by identical or different substituents from the group consisting of CHO, cyano, fluorine, chlorine, bromine, C₁-C₁₂-alkyl, C₁-C₄-alkyl which is mono- to hexasubstituted by identical or different substituents from the group consisting of fluorine and chlorine, C₁-C₁₂-alkoxy, C₁-C₄-alkoxy which is mono- to hexasubstituted by identical or different substituents from the group consisting of fluorine and chlorine, C₁-C₄-alkoxy-C₁-C₄-alkyl, C₁-C₄-alkoxy-ethyleneoxy, C₁-C₄-alkylthio and C₁-C₄-alkylthio which is mono- to hexasubstituted by identical or different substituents from the group consisting of fluorine and chlorine.

4. Compounds of the formula (I) according to Claim 1, in which
Ar¹ represents phenyl which is mono- to trisubstituted by identical or different substituents from the group consisting of fluorine and chlorine and
Ar² represents the radical 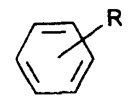 in which
R represents phenyl which is optionally mono- to pentasubstituted by identical or different substituents from the group consisting of CHO, cyano, fluorine, chlorine, bromine, C₁-C₁₂-alkyl, C₁-C₄-alkyl which is mono- to hexasubstituted by identical or different substituents from the group consisting of fluorine and chlorine, C₁-C₁₂-alkoxy, C₁-C₄-alkoxy which is mono- to hexasubstituted by identical or different substituents from the group consisting of fluorine and chlorine, C₁-C₄-alkoxy-C₁-C₄-alkyl, C₁-C₄-alkoxy-ethyleneoxy, C₁-C₄-alkylthio and C₁-C₄-alkylthio which is mono- to hexasubstituted by identical or different substituents from the group consisting of fluorine and chlorine.

5. Compounds of the formula (I) according to Claim 1 in which
Ar¹ represents phenyl which is mono- or disubstituted by identical or different substituents from the group consisting of fluorine and chlorine and
Ar² represents the radical 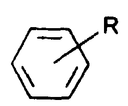 in which
R represents phenyl which is optionally mono- to trisubstituted by identical or different substituents from the group consisting of CHO, cyano, fluorine, chlorine, bromine, C₁-C₈-alkyl, C₁-C₄-alkyl which is mono- to hexasubstituted by identical or different substituents from the group consisting of fluorine and chlorine, C₁-C₈-alkoxy, methoxy or ethoxy, each of which is mono- to pentasubstituted by identical or different substituents from the group consisting of fluorine and chlorine, C₁-C₄-alkoxy-C₁-C₄-alkyl, C₁-C₄-alkylthio, methylthio or ethylthio, each of which is mono- to pentasubstituted by identical or different substituents from the group consisting of fluorine and chlorine.

6. Process for preparing compounds of the formula (I) according to Claim 1, characterized in that
a) hydrazides of the formula (II) 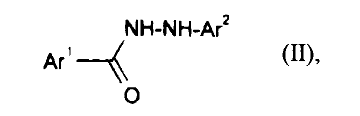 in which
Ar¹ and Ar² are each as defined in Claim 1
are reacted with an acylating agent in the presence of a diluent and, if appropriate, in the presence of a base,
or
b) 3-phenyl-1,3,4-oxadiazolin-2-ones of the formula (III) 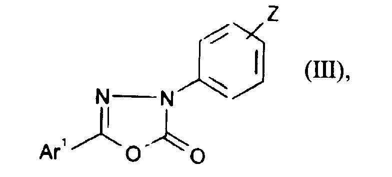 in which
Ar¹ is as defined above and
Z represents a leaving group
are reacted with boronic acids of the formula (IV)
R-B(OH)₂ (IV),
in which
R represents optionally substituted phenyl
in the presence of a base, if appropriate in the presence of a catalyst and in the presence of a diluent.

7. Compounds of the formula (IIIa) 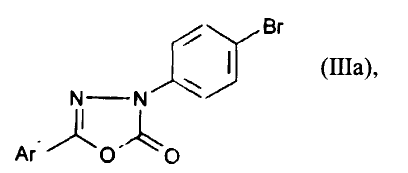 in which
Ar¹ is as defined in Claim 1.

8. Pesticides, characterized in that they comprise at least one compound of the formula (I) according to Claim 1.

9. Use of compounds of the formula (I) according to Claim 1 for controlling pests.

10. Method for controlling pests, characterized in that compounds of the formula (I) according to Claim 1 are allowed to act on pests and/or their habitat.

11. Process for preparing pesticides, characterized in that compounds of the formula (I) according to Claim 1 are mixed with extenders and/or surfactants.

12. Use of compounds of the formula (I) according to Claim 1 for preparing pesticides.

## Revendications

1. Composés de formule (I) 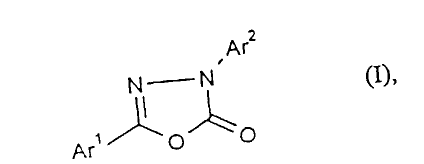 dans laquelle
Ar¹ est un groupe phényle éventuellement substitué et
Ar² est un groupe biphényle éventuellement substitué.

2. Composés de formule (I) suivant la revendication 1, dans laquelle
Ar¹ est un groupe phényle qui est éventuellement substitué une à cinq fois identiques ou différentes par un halogène, un radical alkyle en C₁ à C₆, alkoxy en C₁ à C₆, alkylthio en C₁ à C₆, halogénalkyle en C₁ à C₆, halogénalkoxy en C₁ à C₆, halogénalkylthio en C₁ à C₆, nitro ou cyano et
Ar² représente le reste 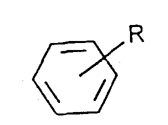 dans lequel
R est un groupe phényle éventuellement substitué une à cinq fois identiques ou différentes par un radical CHO, cyano, halogéno, alkyle en C₁ à C₁₂, halogénalkyle en C₁ à C₄, alkoxy en C₁ à C₁₂, halogénalkoxy en C₁ à C₆, (alkoxy en C₁ à C₆)-(alkyle en C₁ à C₆), (alkoxy en C₁ à C₆)-éthylène-oxy, alkylthio en C₁ à C₆ ou halogénalkylthio en C₁ à C₆.

3. Composés de formule (I) suivant la revendication 1, dans laquelle
Ar¹ est un groupe phényle qui est substitué, le cas échéant, une à trois fois identiques ou différentes par du fluor, du chlore, du brome, un radical alkyle en C₁ à C₄, alkoxy en C₁ à C₄, alkylthio en C₁ à C₄, un radical alkyle en C₁ ou C₂ substitué une à cinq fois identiques ou différentes par du fluor ou du chlore, un radical alkoxy en C₁ à C₄ substitué une à cinq fois identiques ou différentes par du fluor ou du chlore, un radical SCF₃, SCHF₂, nitro ou cyano et
Ar² représente le reste 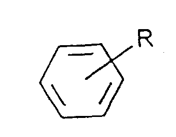 dans lequel
R est un groupe phényle éventuellement substitué une à cinq fois identiques ou différentes par un radical CHO, cyano, fluoro, chloro, bromo, alkyle en C₁ à C₁₂, un radical alkyle en C₁ à C₄ substitué une à six fois identiques ou différentes par du fluor ou du chlore, un radical alkoxy en C₁ à C₁₂, un radical alkoxy en C₁ à C₄ substitué une à six fois identiques ou différentes par du fluor ou du chlore, un radical (alkoxy en C₁ à C₄)-(alkyle en C₁ à C₄), (alkoxy en C₁ à C₄)-éthylène-oxy, alkylthio en C₁ à C₄ ou un radical alkylthio en C₁ à C₄ substitué une à six fois identiques ou différentes par du fluor ou du chlore.

4. Composés de formule (I) suivant la revendication 1, dans laquelle
Ar¹ est un groupe phényle substitué une à trois fois identiques ou différentes par du fluor ou du chlore et
Ar² représente le reste 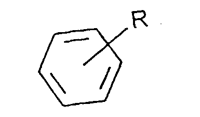 dans lequel
R est un groupe phényle éventuellement substitué une à cinq fois identiques ou différentes par un radical CHO, cyano, fluoro, chloro, bromo, alkyle en C₁ à C₁₂, un radical alkyle en C₁ à C₄ substitué une à six fois identiques ou différentes par du fluor ou du chlore, un radical alkoxy en C₁ à C₁₂, un radical alkoxy en C₁ à C₄ substitué une à six fois identiques ou différentes par du fluor ou du chlore, (alkoxy en C₁ à C₄)-(alkyle en C₁ à C₄), (alkoxy en C₁ à C₄)-éthylène-oxy, alkylthio en C₁ à C₄ ou alkylthio en C₁ à C₄ substitué une à six fois identiques ou différentes par du fluor ou du chlore.

5. Composés de formule (I) suivant la revendication 1, dans laquelle
Ar¹ est un groupe phényle substitué une ou deux fois identiques ou différentes par du fluor ou du chlore et
Ar² représente le reste 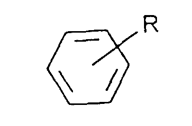 dans lequel
R est un groupe phényle éventuellement substitué une à trois fois identiques ou différentes par un radical CHO, cyano, fluoro, chloro, bromo, alkyle en C₁ à C₈, un radical alkyle en C₁ à C₄ substitué une à six fois identiques ou différentes par du fluor ou du chlore, un radical alkoxy en C₁ à C₈, un radical méthoxy ou éthoxy substitué dans chaque cas une à cinq fois identiques ou différentes par du fluor ou du chlore, un radical (alkoxy en C₁ à C₄)-(alkyle en C₁ à C₄), alkylthio en C₁ à C₄, un radical méthylthio ou éthylthio portant chacun un à cinq substituants fluoro ou chloro identiques ou différents.

6. Procédé de production de composés de formule (I) suivant la revendication 1, caractérisé en ce que
a) on fait réagir des hydrazides de formule (II) 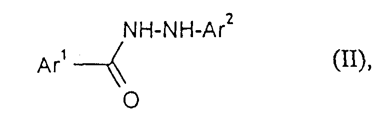 dans laquelle
Ar¹ et Ar² ont la définition indiquée dans la revendication 1,
avec un agent acylant en présence d'un diluant et, le cas échéant, en présence d'une base,
ou bien
b) on fait réagir des 3-phényl-1,3,4-oxadiazoline-2-one de formule (III) 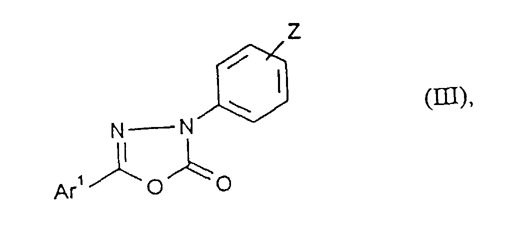 dans laquelle
Ar¹ a la définition indiquée ci-dessus et
z est un groupe partant
avec des acides dérivés du bore de formule (IV)
R-B(OH)₂ (IV)
dans laquelle
R est un groupe phényle éventuellement substitué
en présence d'une base, le cas échéant en présence d'un catalyseur et en présence d'un diluant.

7. Composés de formule (IIIa) 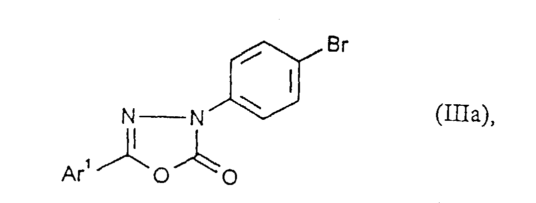 dans laquelle
Ar¹ a la définition indiquée dans la revendication 1.

8. Compositions pesticides, caractérisées par une teneur en au moins un composé de formule (I) suivant la revendication 1.

9. Utilisation de composés de formule (I) suivant la revendication 1 pour combattre des parasites.

10. Procédé pour combattre des parasites, caractérisé en ce qu'on fait agir des composés de formule (I) suivant la revendication 1 sur les parasites et/ou sur leur milieu.

11. Procédé de préparation de compositions pesticides, caractérisé en ce qu'on mélange des composés de formule (I) suivant la revendication 1 avec des diluants et/ou des agents tensio-actifs.

12. Utilisation de composés de formule (I) suivant la revendication 1 pour la préparation de compositions pesticides.
